Europäisches Patentamt

European Patent Office

Office européen des brevets

(11) Veröffentlichungsnummer: **0 407 534 B1**

(12) **EUROPÄISCHE PATENTSCHRIFT**

(45) Veröffentlichungstag der Patentschrift: **23.11.94**

(51) Int. Cl.5: **A61B 5/00**

(21) Anmeldenummer: **90901530.7**

(22) Anmeldetag: **23.01.90**

(86) Internationale Anmeldenummer:
**PCT/CH90/00014**

(87) Internationale Veröffentlichungsnummer:
**WO 90/08501 (09.08.90 90/19)**

(54) **BIOTELEMETRIE-VERFAHREN ZUR ÜBERTRAGUNG VON BIOELEKTRISCHEN POTENTIALDIFFERENZEN, UND VORRICHTUNG ZUR ÜBERTRAGUNG VON EKG-SIGNALEN.**

(30) Priorität: **27.01.89 CH 255/89**

(43) Veröffentlichungstag der Anmeldung:
**16.01.91 Patentblatt 91/03**

(45) Bekanntmachung des Hinweises auf die
Patenterteilung:
**23.11.94 Patentblatt 94/47**

(84) Benannte Vertragsstaaten:
**AT BE CH DE DK ES FR GB IT LI LU NL SE**

(56) Entgegenhaltungen:
**DE-A- 2 818 930**
**US-A- 3 646 606**
**US-A- 3 910 257**

**J.G. Webster : "Encyclopedia of Medical Devices and Instrumen- tation vol. 2", 1988, Wiley and Sons, New York (US), pages 1002-1017**

(73) Patentinhaber: **MEDESE AG**
**Mainaustrasse 15**
**CH-8008 Zürich (CH)**

(72) Erfinder: **GUGGENBÜHL, Walter**
**Glärnischstrasse 40**
**CH-8712 Stäfa (CH)**
Erfinder: **GROGG, Fritz**
**Kreuzstrasse 35**
**D-8008 Zürich (CH)**

(74) Vertreter: **EGLI-EUROPEAN PATENT ATTORNEYS**
**Horneggstrasse 4**
**Postfach 473**
**CH-8034 Zürich (CH)**

Rank Xerox (UK) Business Services
(3.10/3.09/3.3.3)

ELEKTRONIK, Vol. 34, No 1, 11 January 1985, München DE pages 58-60; J. Petsch : "Patientendaten-Erfassung über Lichtleiter kabel"

J.G.Webster : "Encyclopedia of Medical Devices and Instrumentation";1988, Wiley and Sons, New York, Seiten 409-422, "Biotelemetry".

## Beschreibung

Die Erfindung betrifft ein Biotelemetrie-Verfahren zur Übertragung von als bioelektrischen Potentialdifferenzen erzeugten Elektrokardiogramm-Signalen (EKG-Signalen) zu einem zwölf Standardableitungen liefernden EKG-Gerät, wobei man mindestens zehn EKG-Elektroden am Patienten auf zum Erhalt der Standardableitungen übliche Weise ("Arm links, Arm rechts, Fuss links, Fuss rechts, Brustwand Position 1 bis 6") anbringt, wobei die Übertragung der EKG-Signale als optische Einweg-Übertragung vom Patienten zum EKG-Gerät hin erfolgt, während keine elektrische Verbindung zwischen den Elektroden und dem EKG-Gerät besteht, und die im EKG-Gerät aufzuzeichnenden Potentialdifferenzen aus der Differenz zwischen zwei übertragenen Signalen gewonnen werden, gemäss dem Oberbegriff des Anspruchs 1.

Ebenfalls betrifft die Erfindung eine Vorrichtung zur Durchführung dieses Verfahrens in einem EKG-System, das mindestens zehn an einem Patienten angebrachte EKG-Elektroden ("Arm links, Arm rechts, Fuss links, Fuss rechts, Brustwand Position 1 bis 6") und ein EKG-Gerät für zwölf Standardableitungen umfasst, wobei zwei bestimmte EKG-Elektroden ("Fuss links, Fuss rechts") als erste ("Fuss links") und zweite ("Fuss rechts") EKG-Referenzelektroden und die übrigen EKG-Elektroden als EKG-Signalelektroden definiert sind, mit:

einem Senderteil, einem Empfängerteil und mindestens einer dazwischenliegenden Lichtstrecke,

an sich in EKG-Systemen bekannten Eingangsschutz-Schaltungen, an die je eine zugeordnete EKG-Elektrode anschliessbar ist,

an sich in EKG-Systemen bekannten Rechenschaltungen zur Gewinnung der Differenz zwischen Signalen aus zwei Eingangsschutz-Schaltungen, von denen die eine einer EKG-Signalelektrode ("Arm links, Arm rechts, Brustwand Position 1 bis 6") und die andere der ersten EKG-Referenzelektrode ("Fuss links") zugeordnet ist, so dass auch jede Signaldifferenz einer EKG-Signalelektrode zugeordnet ist, mindestens einen modulierbaren Lichtsender und mindestens einen Lichtempfänger mit einem Ausgang zur Abgabe der Modulation eines empfangenen Lichtstrahls, gemäss dem Oberbegriff des Anspruchs 5.

Ein Elektrokardiogramm (EKG) ist die Aufzeichnung des Zeitverlaufs von Herzaktionsspannungen bei einem Lebewesen, im allgemeinen bei einem menschlichen Patienten. Für weitere Definitionen wird auf DIN 13401 (Januar 1953) verwiesen.

Die Analyse des EKGs liefert dem Arzt wichtige Hinweise über die Herzfunktion des Patienten. Die Annahme der EKG-Signale erfolgt an EKG-Elektroden, die am Patienten angebracht und auf zum Erhalt der sogenannten Standardableitungen übliche Weise ("Arm links, Arm rechts, Fuss links, Fuss rechts, Brustwand Position 1 bis 6") am Patienten angeordnet werden.

Am Patienten hängen dabei bis zu zehn Kabel, welche die EKG-Elektroden mit dem EKG-Gerät verbinden, in welchem die EKG-Signale verarbeitet und aufgezeichnet werden. Diese Kabel behindern den Patienten und erschweren die Aufnahme von Belastungs-EKG, beispielsweise wenn untersucht wird, wie das Herz des Patienten auf physische Arbeit auf einem Ergometer reagiert: der Patient hat nur sehr beschränkte Bewegungsmöglichkeiten, nicht zuletzt weil die EKG-Elektroden abfallen, wenn bedingt durch das Gewicht und die Steifigkeit der Kabel daran gerissen wird oder sich der Patient heftig bewegt. Ebenfalls nicht zu übersehen ist die psychische Belastung des Patienten, der über elektrische Leitungen mit einem am Netz betriebenen Gerät verbunden ist und darauf mit Angst vor einem elektrischen Schlag reagiert. Der Effekt dieser psychischen Belastung kann sich mit dem Effekt der echten physischen Belastung kombinieren, was dann die Untersuchung der Herzfunktion verfälscht.

Es besteht daher ein Bedarf für eine jedenfalls nicht-elektrische und gegebenenfalls immaterielle ("drahtlose") Verbindung zwischen den am Patienten angebrachten EKG-Elektroden und dem EKG-Gerät: diese nichtelektrische Verbindung soll die übliche Verbindung mittels elektrischer Leitungen ohne weiteres ersetzen. In anderen Worten, es besteht ein Bedarf für ein an die Gegebenheiten der EKG-Aufzeichnung angepasstes nicht-elektrisches Übertragungsverfahren für EKG-Signale von den an einem Patienten angebrachten EKG-Elektroden zum EKG-Gerät, das die EKG-Signale verarbeitet und aufzeichnet.

Es besteht auch ein Bedarf für eine Vorrichtung, an deren Eingängen die am Patienten angebrachten EKG-Elektroden genau so angeschlossen werden, als ob sie am EKG-Gerät angeschlossen wären, und deren Ausgänge an die Eingänge des EKG-Geräts genau so angeschlossen werden, wie wenn es die am Patienten angebrachten EKG-Elektroden wären. Über diese gewünschte Vorrichtung sollen üblich ausgebildete und angeordnete EKG-Elektroden mit einem üblichen EKG-Gerät verbunden werden, wobei die Verbindung nicht-elektrisch und gegebenenfalls immateriell sein soll.

Unter diesen Bedingungen wird zwischen den EKG-Elektroden und dem EKG-Gerät eine Verbindung mittels elektromagnetischer Wellen nahegelegt. Eine solche Verbindung erfordert einen Senderteil, eine Übertragungsstrecke und einen Empfängerteil. Der Senderteil ist dabei vom Patienten zu tragen, sonst wäre ja der Patient mit dem Senderteil über Elektrodenkabel verbunden, was als widersprüchlich und unzweckmässig zurückzuweisen ist. Die zu beseitigende Behinderung des Patienten wird aber offensichtlich erst dann aufgehoben, wenn der Senderteil vom Patienten auch leicht tragbar ist. Der Senderteil hat zudem die üblichen Auflagen bezüglich der Patientensicherheit und der Defibrillationsfestigkeit zu erfüllen (Defibrillation ist eine Massnahme gegen Herzflimmern). Ausserdem muss die betrachtete Verbindung einen grossen Datenfluss übertragen können.

Allgemein sind Telemetriesysteme für Biosignale bereits bekannt. Aus dem Artikel von N.Kudo, K.Shimizu und G.Matsumoto "Optical biotelemetry using indirect light transmission" auf Seiten 55-58 von "Biotelemetry IX" (1987, herausgegeben von H.P. Kimmich und M.R.Neuman) kennt man die Übertragung von bioelektrischen Signalen von an einem Patienten angebrachten Elektroden zu einem Auswertegerät, insbesondere einem EKG-Gerät, das die Signale verarbeitet und aufzeichnet. Dabei erfolgt die Übertragung der Signale optisch, gegebenenfalls im infraroten Bereich, als Einweg-Übertragung von den Elektroden zum Auswertegerät hin, und es wird keine elektrische Verbindung zwischen den Elektroden und dem Auswertegerät erstellt.

Nachteilig ist bei dieser bekannten Übertragungsweise, dass die Messwerte als Analogwerte übertragen werden: die am Patienten gewonnenen analogen Messwerte werden als Pulsintervalle moduliert, gewandelt, multiplexiert, übertragen, demultiplexiert und demoduliert, so dass schliesslich wieder Analogwerte zur Verfügung stehen. Eine solche Vorgehensweise erlaubt nicht, moderne Techniken zur Verminderung der Störanfälligkeit der Übertragung und gegebenenfalls zur Korrektur von Übertragungsfehlern anzuwenden, welche auf dem Gebiet der Übertragung von digitalen Daten bekannt sind.

Insbesondere wird diese bekannte Übertragungsweise durch das Licht von Wechselstrom-Lichtquellen, d.h. durch den 50-Hz-Brumm und die 100-Hz-Störung durch Leuchtstoffröhren wie auch noch durch deren Oberschwingungen, empfindlich gestört.

Ausserdem ist bei dieser bekannten Übertragungsweise nachteilig, dass die übertragenen Analogwerte keineswegs an die Gegebenheiten der EKG-Aufzeichnung mit einem die zwölf Standardableitungen liefernden EKG-Gerät angepasst sind und somit das bekannte optische Übertragungssystem nicht ohne weiteres anstelle der üblichen Verbindung mittels elektrischer Leitungen verwendbar ist. Insbesondere werden die übertragenen Werte dem EKG-Gerät nicht zeitgleich zugeführt, weil die zur Multiplexierung nötige Abtastung ("sampling") auf den verschiedenen Kanälen gegenseitig zeitverschobene Signale liefert. Die nötige Korrektur dieser Zeitverschiebung ist bei der bekannten Übertragungsweise nicht vorgesehen.

Ferner ist bei dieser bekannten Übertragungsweise nachteilig, dass für jede der den Arzt interessierenden Potentialdifferenzen zwischen spezifisch ausgewählten Elektroden ein Kanal des Telemetriesystems benötigt wird, so dass die Anzahl von aufgezeichneten Potentialdifferenzen gleich der Anzahl von Übertragungskanälen bzw. von übertragenen Signalen ist. In dem im zitierten Artikel beschriebenen System sind nur drei Kanäle für Potentialdifferenzen entsprechende Signale vorgesehen (der vierte Kanal ist einem der Temperatur entsprechenden Signal zugeordnet). Bei Verwendung dieser Lehre in Systemen, zur Aufzeichnung einer grösseren Anzahl von Potentialdifferenzen bestimmt sind, wie z.B. in einem EKG-System mit zwölf Standardableitungen, wären zwölf Übertragungskanäle erforderlich.

Ein Verfahren und eine Vorrichtung der eingangs erwähnten Art sind beispielsweise aus J.G. Webster "Encyclopedia of Medical Devices and Instrumentation" (Wiley & Sons, New York, 1988), insbesondere in dem von T.H. Stanley geschriebenen Kapitel "Electrocardiography" auf Seiten 1002-1017, bekannt.

In diesem Dokument wird offenbart, dass für jede EKG-Signalelektrode die Differenz zwischen dem daran abgenommenen Signal und dem einer EKG-Referenzelektrode ("Fuss links") abgenommenen Signal gebildet und übertragen wird. Es werden 10 EKG-Elektroden LL ("Fuss links"), RL ("Fuss rechts"), LA ("Arm links"), RA ("Arm rechts") und $v_1$ bis $v_6$ ("Brustwand Position 1 bis 6") verwendet. Mit LL ("Fuss links") als Referenzelektrode für die Bestimmung der Potentiale der anderen 9 Elektroden ergeben sich somit 9 Signale. Das Signal aus RL ("Fuss rechts") wird dabei nicht direkt zur EKG-Aufnahme verwendet, sondern zur Unterdrückung des Brumms ("common mode rejection"). Die 9 zu übertragenden Signale werden wie folgt definiert:

$$I = LA - RA$$
$$II = LL - RA$$
$$III = LL - LA$$
$$V_1 = v_1 - (LA + RA + LL) / 3$$
$$V_2 = v_2 - (LA + RA + LL) / 3$$
$$V_3 = v_3 - (LA + RA + LL) / 3$$
$$V_4 = v_4 - (LA + RA + LL) / 3$$
$$V_5 = v_5 - (LA + RA + LL) / 3$$
$$V_6 = v_6 - (LA + RA + LL) / 3$$

Die Übertragung dieser 9 Signale erfolgt beispielsweise optisch, um den Patienten elektrisch vom EKG-Gerät zu isolieren. Das zehnte Signal aus RL ("Fuss rechts") erscheint in diesen Gleichungen nicht, es wird aber gleichwohl berücksichtigt, sonst könnte der Brumm nicht unterdrückt werden. Zur Unterdrückung des Brumms wird das patientenseitige Potential RL ("Fuss rechts") dem empfangsseitigen Referenzpotential gleichgesetzt. Der Patient ist also mit dem EKG-Gerät elektrisch verbunden.

Aus US-A-3910257 ist die Übertragung von EKG-Signalen zwischen einem Patienten und einem EKG-Gerät bekannt, bei welcher keine elektrische Verbindung zwischen dem Patienten und dem EKG-Gerät besteht. Am Patienten ist eine Mehrzahl von EKG-Elektroden angebracht, mit denen EKG-Signale als bioelektrische Potentialdifferenzen erzeugt werden. Die EKG-Signale werden unter der Kontrolle einer patientenseitigen Steuerungseinrichtung multiplexiert, das Multiplex-Signal wird analog/digital gewandelt, und das digitale Signal wird über ein Koaxialkabel zum EKG-Gerät übertragen. Das im EKG-Gerät empfangene Signal wird demoduliert, verstärkt, demultiplexiert und digital/analog gewandelt, um EKG-Signale zu ergeben, die auf einem Bildschirm angezeigt werden. Das ganze Biotelemetrie-Verfahren funktioniert als Zweiweg-Übertragung zwischen dem Patienten und dem EKG-Gerät, denn das EKG-Gerät sendet über einen zweiten Übertragungsweg Signale, von denen die patientenseitige Steuerungseinrichtung selbst gesteuert wird. Auf beiden Übertragungswegen wird mittels Optokoppler dafür gesorgt, dass die elektrische Verbindung zwischen den Elektroden und dem EKG-Gerät unterbrochen wird.

Die Lehre aus US-A-3910257 enthält jedoch keinen Hinweis auf eine besondere Verarbeitung der EKG-Signale und insbesondere keinen Hinweis auf eine Unterdrückung des Brumms.

Aufgabe der Erfindung ist es daher, bei einem Biotelemetrie-Verfahren der eingangs erwähnten Art mit acht Kanälen zur Übertragung von Elektrokardiogramm-Signalen (EKG-Signalen) zu einem zwölf Standardableitungen liefernden EKG-Gerät die Nachteile der bekannten Übertragungsweise zu überwinden und insbesondere die benötigte Anzahl von Übertragungskanälen zu vermindern.

Erfindungsgemäss wird diese Aufgabe dadurch gelöst, dass man

zwei bestimmte EKG-Elektroden ("Fuss links, Fuss rechts") als erste ("Fuss links") und zweite ("Fuss rechts") EKG-Referenzelektroden und die übrigen EKG-Elektroden ("Arm links, Arm rechts, Brustwand Position 1 bis 6") als EKG-Signalelektroden definiert,

die zweite EKG-Referenzelektrode ("Fuss rechts") zur Bestimmung eines patientenseitigen Massepotentials verwendet,

für jede EKG-Signalelektrode die Differenz zwischen dem daran abgenommenen EKG-Signal und dem an der ersten EKG-Referenzelektrode ("Fuss links") abgenommenen EKG-Signal bildet und somit ein zu übertragendes Signal erzeugt, das vom gemeinsamen Gleichspannungsanteil und vom Brumm befreit ist ("common mode rejection"),

die zu übertragenden Signale einweg-seriell überträgt, indem man sie multiplexiert, das Multiplex-Signal analog/digital wandelt und danach in ein codiertes Signal umsetzt, das gegebenenfalls mit Fehlerschutz-Zusatzinformation versehen wird, mit dem codierten Signal einen Lichtstrahl moduliert, den Lichtstrahl sendet, überträgt und empfängt, den empfangenen Lichtstrahl demoduliert, die gewonnene Modulation decodiert, das decodierte Signal gegebenenfalls nach Vornahme einer Korrektur mit Hilfe der Fehlerschutz-Zusatzinformation digital/analog wandelt und demultiplexiert, um übertragene EKG-Signale zu erhalten,

und die übertragenen EKG-Signale den entsprechenden Eingängen ("Arm links, Arm rechts, Brustwand Position 1 bis 6") des EKG-Geräts zuführt, während man die übrigen Eingänge des EKG-Geräts ("Fuss rechts, Fuss links") mit einer Masse des EKG-Geräts verbindet.

Die analog/digital gewandelten Signale werden vorzugsweise im Manchester-Code oder als Frequenzsprung-Modulation codiert und vorzugsweise mit einer Fehlerschutz-Zusatzinformation versehen.

Vorzugsweise erfolgt die Übertragung optisch, wobei sie über einen Lichtleiter geführt wird.

Eine Vorrichtung zur Durchführung des erfindungsgemässen Verfahrens in einem EKG-System, das mindestens zehn an einem Patienten angebrachte EKG-Elektroden ("Arm links, Arm rechts, Fuss links, Fuss rechts, Brustwand Position 1 bis 6") und ein EKG-Gerät für zwölf Standardableitungen umfasst, wobei zwei bestimmte EKG-Elektroden ("Fuss links, Fuss rechts") als erste ("Fuss links") und zweite ("Fuss rechts") EKG-Referenzelektroden und die übrigen EKG-Elektroden als EKG-Signalelektroden definiert sind, mit:

einem Senderteil, einem Empfängerteil und mindestens einer dazwischenliegenden Lichtstrekke,

an sich in EKG-Systemen bekannten Eingangsschutz-Schaltungen, an die je eine zugeordnete EKG-Elektrode anschliessbar ist,

an sich in EKG-Systemen bekannten Rechenschaltungen zur Gewinnung der Differenz zwischen Signalen aus zwei Eingangsschutz-Schaltungen, von denen die eine einer EKG-Signalelektrode ("Arm links, Arm rechts, Brustwand Position 1 bis 6") und die andere der ersten EKG-Referenzelektrode ("Fuss links") zugeordnet ist, so dass auch jede Signaldifferenz einer EKG-Signalelektrode zugeordnet ist, mindestens einen modulierbaren Lichtsender und mindestens einen Lichtempfänger mit einem Ausgang zur Abgabe der Modulation eines empfangenen Lichtstrahls,

ist erfindungsgemäss gekennzeichnet durch eine Verbindung des Ausgangs der Eingangsschutz-Schaltung, die der zweiten EKG-Referenzelektrode ("Fuss rechts") zugeordnet ist, mit einer gemeinsamen Masseleitung der Rechenschaltungen,

einen im Senderteil angeordneten Multiplexer für die gewonnenen Signaldifferenzen, dessen Ausgang an einen Modulationseingang des Lichtsenders über die Reihenschaltung eines Analog/Digital-Wandlers, eines Codierers und gegebenenfalls einer Schaltung zum Zufügen von Fehlerschutz-Zusatzinformation angeschlossen ist, und

einen im Empfängerteil angeordneten Demultiplexer mit einem Eingang, der an den Ausgang des bzw. der Lichtempfänger gegebenenfalls über eine Schaltung zur Vornahme einer Korrektur mit Hilfe der Fehlerschutz-Zusatzinformation und dann über die Reihenschaltung eines Decodierers und eines Digital/Analog-Wandlers angeschlossen ist, und mit Ausgängen, von denen jeder einer der im Senderteil gebildeten Signaldifferenzen zugeordnet ist und ein Signal führt, das einem entsprechenden Eingang ("Arm links, Arm rechts, Brustwand Position 1 bis 6") des EKG-Geräts zuführbar ist.

In einer bevorzugten Ausbildung der erfindungsgemässen Vorrichtung ist der Senderteil als selbständiges Gerät ausgebildet, das vom Patienten tragbar ist. Dabei sind vorzugsweise mindestens ein und vorzugsweise mehrere Lichtsender an mindestens einer dem Patienten nicht zugewendeten Seite des Senderteils und mindestens ein und vorzugsweise mehrere Lichtempfänger an einem bzw. mehreren festen Stellen eines den Patienten umgebenden Raumes angeordnet.

In einer weiteren bevorzugten Ausbildung der erfindungsgemässen Vorrichtung ist der Senderteil als selbständiges zweiteiliges Gerät mit einem Betriebsteil und einem Lichtsender-Tragteil ausgebildet, wobei die beiden Teile vom Patienten tragbar sind und der Lichtsender-Tragteil mindestens ein und vorzugsweise mehrere Lichtsender an mindestens einer dem Patienten nicht zugewendeten Seite aufweist, während mindestens ein und vorzugsweise mehrere Lichtempfänger an einem bzw. mehreren festen Stellen eines den Patienten umgebenden Raumes angeordnet sind.

Bei der Erfindung wird die Möglichkeit erkannt und genutzt, in der EKG-Technik die 12 Standardableitungen aus 8 gegenüber einer Referenzelektrode gewonnenen Signalen zum einen Teil direkt, zum anderen Teil durch Bildung der Differenz zwischen zwei Signalen im Auswertegerät zu bilden. Die Anzahl der darstellbaren "Ableitungen" könnte nach diesem Verfahren, falls für den Mediziner von Interesse, bis auf 36 erhöht werden.

In der erfindungsgemässen Vorrichtung wird diese Art der Erfassung und Übertragung von bioelektrischen Potentialdifferenzen im Zusammenhang mit einem EKG-System durch die senderseitige Verwendung von zwei Referenzelektroden erreicht, von denen die eine ("Fuss links") als Referenzelektrode in obigem Sinn und die andere ("Fuss rechts") zur Festlegung des elektrischen Massepotentials des Senderteils verwendet wird.

Beim erfindungsgemässen Verfahren werden einzelne "Ableitungen" aus der Differenz zwischen je einem Paar von übertragenen Potentialwerten berechnet, was eine höhere Genauigkeit der Übertragungskanäle erfordert als bei der herkömmlichen direkten Übertragung von den "Ableitungen" entsprechenden Potentialwerten. Beispielsweise sind dabei Differenzen von zwei nahezu gleich grossen Potentialwerten zu bilden, was die relative Genauigkeit des Resultats beeinträchtigt. Die benötigte höhere Genauigkeit der Übertragungskanäle wird erfindungsgemäss durch digitale Übertragung mit einer genügenden Anzahl Bits sichergestellt und wäre in einem herkömmlichen System, z.B. mit dem im zitierten Artikel beschriebenen analogen Übertragungsverfahren, nur unter grossem apparativem Aufwand (Kosten, Gewicht) erreichbar.

Zur Reduktion des Gewichts des Senderteils und des allgemeinen Aufwands arbeitet das erfindungsgemässe Verfahren wie auch das aus "Biotelemetry IX" bekannte Verfahren mit einer Einweg-Übertragung ohne Empfangsrückmeldung. Zur Erreichung der höchstmöglichen Übertragungsgeschwindigkeit und Datensicherheit bei möglichst geringer Störanfälligkeit erfolgt die Übertragung beim erfindungsgemässen Verfahren digital und nach einem geeigneten Codierverfahren codiert, vorzugsweise im Manchester-Code oder als Frequenzsprung-Modulation ("frequency shift keying" = FSK). Diese Codierverfahren haben insbesondere den Vorteil, dass ihre Frequenzspektren keinen kontinuierlichen und keinen wirksamen niederfrequenten Anteil haben, so dass der 50-Hz-Brumm und die 100-Hz-Störung durch Leuchtstoffröhren wie auch noch deren Oberschwingungen unterdrückt werden können. Ausserdem erfordern der Manchester-Code und die FSK-Modulation keine Synchronisierung des Takts beim Empfang mit dem Takt beim Senden, denn sie sind beim Empfang selbstsynchronisierend und gewährleisten somit beim Empfang die Datensicherheit und geringe Störanfälligkeit trotz Einweg-Übertragung ohne Empfangsrückmeldung.

Ausserdem erlaubt die Übertragung von digitalen Daten, diesen eine Fehlerschutz-Zusatzinformation beizufügen, z.B. zur zyklischen Redundanzprüfung ("cyclic redundancy check" = CRC) zur Paritätsprüfung oder nach dem Hamming-Code. Schaltungen zur senderseitigen Bildung der Fehlerschutz-Zusatzinformation und zur empfängerseitigen Durchführung der entsprechenden Prüfungen sind dem Fachmann wohlbekannt.

Die erfindungsgemässe Vorrichtung ist dazu bestimmt und ausgebildet, ohne weiteres an die Stelle der üblichen Verbindungskabel zu treten, der Arzt muss nichts umlernen oder dazulernen, wenn er die erfindungsgemässe Vorrichtung verwendet. Der Senderteil greift die EKG-Elektroden ab, bildet die EKG-Signale, verstärkt sie, wandelt sie wie benötigt und übermittelt sie auf optische Weise und gegebenenfalls draht- bzw. kabellos. Der Empfängerteil bildet wieder die EKG-Signale und schwächt sie auf den normalen Wert von reellen, direkt an EKG-Elektroden abgegriffenen EKG-Signalen ab, so dass man die Ausgänge des Empfängerteils direkt an die entsprechenden Eingänge eines üblichen EKG-Geräts anschliessen kann. Das EKG-Gerät "bemerkt" keinen Unterschied, ob nun der Patient direkt über die üblichen Kabel oder über die erfindungsgemässe Vorrichtung daran angeschlossen ist, und die dabei erhaltene EKG-Aufzeichnung ist in beiden Fällen die gleiche.

Ausserdem hat die beim erfindungsgemässen Verfahren und in der erfindungsgemässen Vorrichtung verwendete optische Übertragung den Vorteil, keinen gesetzlichen Vorschriften und Beschränkungen bezüglich Sende- und Empfangslizenz unterworfen zu sein.

Das erfindungsgemässe Verfahren, Weiterbildungen davon und Beispiele von Ausbildungen der erfindungsgemässen Vorrichtung werden nachstehend unter Bezugnahme auf die Zeichnungen näher erläutert. Es zeigen:

Fig. 1 ein Blockschaltbild eines mit einer erfindungsgemässen Vorrichtung versehenen EKG-Systems,

Fig. 2 eine schematisch angegebene Ausbildung einer im Senderteil der erfindungsgemässen Vorrichtung verwendeten Schaltung zum Eingangsschutz,

Fig. 3 eine schematisch angegebene Ausbildung einer im Senderteil der erfindungsgemässen Vorrichtung verwendeten, der Schaltung zum Eingangsschutz nachgeschalteten Schaltung zur Vorverstärkung, Differenzbildung, und "Anti-Aliasing"-Filterung,

Fig. 4 eine schematisch angegebene Ausbildung einer im Empfängerteil der erfindungsgemässen Vorrichtung verwendeten Ausgangsschaltung,

Fig. 5 eine schematisch und perspektivisch angegebene Ausbildung eines mit Lichtsendern versehenen, in der erfindungsgemässen Vorrichtung verwendeten Gehäuses eines Senderteils, und

Fig. 6 eine schematisch und perspektivisch angegebene Ausbildung eines mit Lichtsendern versehenen, in der erfindungsgemässen Vorrichtung verwendeten Stirnbands.

Zur Erläuterung der Erfindung müssen vorerst einige am Eingang der erfindungsgemässen Vorrichtung zu erfüllende Bedingungen diskutiert werden.

Beim elektrischen Übergang zwischen den EKG-Elektroden und der Haut des Patienten findet der Übergang von der Ionenleitung des Körpers zur Elektronenleitung der EKG-Elektrodenanschlüsse statt. Dadurch entsteht eine galvanoelektrische Gleichspannung, die wegen Ungleichheiten der Haut verschiedene Werte annehmen kann und bewirkt, dass auch zwischen zwei EKG-Elektroden eine galvanoelektrische Gleichspannung besteht. Diese ist in der Signalverarbeitung zu unterdrükken, weil sie bedeutend grösser ist als das Nutzsignal von ca. 1 mV. Wenn die galvanoelektrische Gleichspannung wie üblich erst nach einer Vorverstärkung der EKG-Signale im Eingangsverstärker unterdrückt wird, hat dies zur Folge, dass diese Gleichspannungen linear zu verstärken sind.

Weiter soll über die EKG-Elektroden möglichst wenig Strom fliessen, weil dieser die chemische Zusammensetzung der Haut verändert und Polarisationsspannungen hervorruft, die zeitlich sehr stark schwanken können. Die Eingangsströme sollen kleiner bleiben als $0,1\mu A$ und der Eingangsverstärker soll eine hohe Eingangsimpedanz über 2 MΩ aufweisen.

Bedingt durch den Patienten und sein Umfeld treten am Ausgang der EKG-Elektroden neben dem erwünschten Nutzsignal einige überlagerte Störspannungen auf, wobei die grössten davon, nämlich der 50-Hz-Brumm und seine Oberschwingungen, als "common mode"-Spannung auftreten und mit dafür geeigneten, an sich bekannten Techniken unterdrückbar sind: erwünscht ist dabei ein Unterdrückungsfaktor (CMRR, "common mode rejection rate") von 80dB.

Der Frequenzgang von EKG-Signalen ist solcher Art, dass Frequenzen über 100Hz vernachlässigt werden können, während die EKG-Signale innerhalb des Frequenzbands von 0,1Hz bis 100Hz sowohl amplituden- als auch phasenmässig möglichst verzerrungsfrei zu übertragen und zu verarbeiten sind.

Die Eingänge der erfindungsgemässen Vorrichtung bzw. ihre empfindlichen Eingangsverstärker sollen vor Übersteuerung oder Anregung durch hochfrequente Einstreuungen geschützt werden. Andererseits sollen der verbleibende Gleichspannungsanteil und Störungen vom Nutzsignal abgetrennt werden, was mit Hoch- und Tiefpassfiltern verschiedener Zeitkonstanten (etwa 3s und etwa 4ms) erreicht werden kann. Auch sollen die Eingänge der Eingangsverstärker die beim Vornehmen einer Defibrillation auftretenden hohen Spannungsimpulsen (3kV während 5ms) ertragen bzw. davor abgeschirmt werden. Dies wird auf bekannte Weise durch Signalbegrenzung mittels antiseriell geschalteter Zenerdioden erreicht: sogenannte bidirektionale Zenerdioden, die zu solchen Zwecken entwickelt wurden, sind im Handel erhältlich.

Die Sicherheit des Patienten ist auf gesetzlich vorgeschriebene Weise zu gewährleisten, z.B. nach Schweizer SEV-Norm TP62/d, wonach bei einem Versagen der Vorrichtung höchstens 5mA und beim normalen Betrieb höchstens 100µA über den Patienten fliessen dürfen.

Zur Erläuterung der Erfindung müssen auch noch einige von der elektromagnetischen und insbesondere optischen Übertragung gestellte Bedingungen diskutiert werden.

Die elektromagnetische und insbesondere optische Übertragung der EKG-Signale könnte parallel auf mehreren Kanälen oder seriell auf einem einzigen Kanal erfolgen: Überlegungen zur Wirtschaftlichkeit und zur Tragbarkeit des Senderteils führen zur Wahl der seriellen Übertragung auf einem einzigen Kanal, was bedingt, dass die Signale vor der Übertragung multiplexiert und nach der Übertragung demultiplexiert werden. Dann aber muss das Frequenzband des zu multiplexierenden Signals eine obere Grenze aufweisen, die nach dem Theorem von Shannon die Hälfte der Abtastfrequenz des Multiplexers nicht überschreitet: dies wird mit einem sogenannten "Anti-Aliasing"-Filter erreicht, z.B. mit einem Butterworthfilter 2. Ordnung mit einer Grenzfrequenz von 180Hz bei einer Abtastfrequenz von 600Hz am Multiplexer.

Zur seriellen Übertragung ist eine Multiplexierung nötig, herkömmliche Multiplexer sind aber nicht fähig, die EKG-Signale im Millivoltbereich zu verarbeiten, und ihre Eingangsimpedanzen entsprechen nicht den Anforderungen von EKG-Eingangsverstärkern. Auch sind in den EKG-Signalen Gleichspannungsanteile zu unterdrücken, was wie bereits erwähnt mit Filtern erfolgt, deren Zeitkonstante von etwa 3s zu Einschwingzeiten führt, die wesentlich länger sind als die Anschaltzeiten des EKG-Signals am Multiplexer: dieser muss daher nach solchen Filtern angeordnet werden.

Aus diesen Gründen weist die erfindungsgemässe Vorrichtung je einen Eingangsverstärker pro EKG-Signal auf, und die Multiplexierung erfolgt nach den Eingangsverstärkern. Von Vorteil ist dabei die Möglichkeit, Analog/digital-Wandler mit integrierten Multiplexern zu verwenden.

Bei einem herkömmlichen EKG-Gerät besteht die Möglichkeit, mit Differenzverstärkern die momentan benötigten Signale bipolar-differentiell abzugreifen, womit auch die "common mode"-Spannung gegenüber der Referenzmasse ("Fuss rechts") unterdrückt wird. Das bedingt, dass bei Verwendung des erfindungsgemässen Verfahrens und der erfindungsgemässen Vorrichtung die im EKG-Gerät erfolgende Unterdrückung der "common mode"-Spannung nicht zur Unterdrückung der für die Übertragung massgebenden "common mode"-Anteile der momentan ausgewerteten EKG-Signale wirksam ist. Daher muss die "common mode"-Spannung soweit möglich bereits vor der Übertragung der EKG-Signale, vorzugsweise in den Eingangsverstärkern der erfindungsgemässen Vorrichtung erfolgen, um eine Übersteuerung der Übertragungskanäle und batteriebetriebenen Eingangsverstärker zu vermeiden. Deshalb wird deren Masseleitung hochohmig mit dem Patienten verbunden, was mittels der für Untersuchungszwecke nicht direkt verwendeten EKG-Elektrode am rechten Fuss des Patienten erfolgt: diese EKG-Elektrode wird somit zur zweiten EKG-Referenzelektrode ("Fuss rechts") und bestimmt das patientenseitige Massepotential. Empfängerseitig wird der Anschluss für die zweite EKG-Referenzelektrode ("Fuss rechts") nicht mehr benötigt, da er aber vorhanden ist, darf sein Potential nicht unbestimmt bleiben, daher ist er gegebenenfalls hochohmig mit der Masse des Empfängerteils verbunden.

Bei einem auf übliche Weise verwendeten EKG-Gerät mit direkt daran angeschlossenen EKG-Elektroden stehen alle EKG-Signale dem EKG-Gerät parallel zur Verfügung: das EKG-Gerät kann jederzeit auf alle EKG-Signale zugreifen, diese sind auf das Potential der EKG-Elektrode am rechten Fuss des Patienten (Referenzmasse) bezogen.

Bei Verwendung des erfindungsgemässen Verfahrens und der erfindungsgemässen Vorrichtung werden die EKG-Signale hingegen seriell übertragen. Da keine Rückmeldung vom Empfänger zum Sender erfolgt mit der Angabe, welche Signale zu welchem Zeitpunkt vom EKG-Gerät momentan benötigt werden, müssen die EKG-Signale wieder alle parallel bereitgestellt werden. Am einfachsten ist es, alle empfängerseitig bereitgestellten EKG-Signale auf eine einzige gemeinsame Referenzspannung zu beziehen, nämlich auf die Masse des Empfängerteils. Daher wird der empfängerseitige Anschluss für die erste EKG-Referenzelektrode ("Fuss links") nicht mehr benötigt, da er aber vorhanden ist, darf sein Potential nicht unbestimmt bleiben, deshalb ist er gegebenenfalls hochohmig mit der Masse des Empfängerteils verbunden.

In bestehenden EKG-Geräten werden zur Auswertung der EKG-Signale immer echte Differenzverstärker verwendet, denn sie benötigen diese nur in geringer Anzahl, nämlich pro aufgezeichnetes EKG-Signal nur einen Differenzverstärker, denn die Aufzeichnung wird nach Bedarf vom einen auf das andere EKG-Signal umgeschaltet.

Bei Verwendung des erfindungsgemässen Verfahrens und der erfindungsgemässen Vorrichtung wäre, nach dieser üblichen Vorgehensweise, für jedes übertragene EKG-Signal ein Differenzverstärker vorzusehen, dessen beiden Eingängen noch je ein Rechenverstärker ("Buffer") vorzuschalten wäre, um einen sehr hochohmigen Eingang des Verstärkers zu gewährleisten. Es wäre also eine Vielzahl von Differenz- und Rechenverstärkern nötig, was Aufwands- und Gewichtsprobleme bringt. Da aber alle senderseitigen EKG-Signale stets auf die gleiche Referenzspannung der ersten EKG-Referenzelektrode ("Fuss links") bezogen sind, ist es nicht nötig, echte Differenzverstärker zu verwenden. Jedes EKG-Signal kann dem nichtinvertierenden Eingang eines zugeordneten Rechenverstärkers zugeführt werden, da dieser Eingang hochohmig ist. Zur richtigen Bildung der gewünschten Differenzen müssen alle EKG-Signale in ihrem jeweiligen Rechenverstärker gleich verstärkt werden: dies wird durch Einstellung der Verstärkung am anderen, invertierenden Eingang des jeweiligen Rechenverstärkers erreicht. Schliesslich ist der Ausgang des Rechenverstärkers, welcher der ersten EKG-Referenzelektrode ("Fuss links") zugeordnet ist, mit den invertierenden Eingängen der Rechenverstärker verbunden, die den anderen EKG-Signalen zugeordnet sind.

Von Vorteil ist bei dieser Ausbildung der Eingangsverstärker, dass Signale zur Verfügung stehen, mit denen eine Mitführschaltung (sogenanntes "Bootstrap") die Wirkung der Kabelabschirmungen wesentlich erhöhen kann. Da aus praktischen Gründen nicht jeder Eingang einzeln abgeschirmt werden kann, wird der Schirm aller Eingänge auf ein und demselben Potential mitgeführt. Dieses Potential wird in einem gesonderten Rechenverstärker als Mittelwert ausgewählter EKG-Signale gebildet. Weil die Summenbildung an diesem Rechenverstärker invertierend ist, ist noch ein zweiter Rechenverstärker zur Inversion des erhaltenen Schirmsignals vorgesehen.

Die Forderung, dass das EKG-Gerät keinen Unterschied "bemerken" soll, ob nun der Patient direkt über die üblichen Kabel oder über die erfindungsgemässe Vorrichtung daran angeschlossen ist, hat zur Folge, dass die übertragenen Signale in demselben Masse, wie sie senderseitig verstärkt worden sind, empfängerseitig wieder abzuschwächen sind. Im Empfängerteil ist die entsprechende Schnittstelle zwischen den Ausgängen des Demultiplexers und den Eingängen des EKG-Geräts ganz einfach: die EKG-Signale an den Ausgängen des Demultiplexers werden lediglich in einem Ohmschen Spannungsteiler so abgeschwächt, dass sie den normalen Werten von reellen, direkt an EKG-Elektroden abgegriffenen EKG-Signalen entsprechen, damit man sie direkt in die entsprechenden Eingänge eines üblichen EKG-Geräts einspeisen kann. Das Rauschen aus der Digital/Analog-Wandlung wird von den im EKG-Gerät ohnehin vorhandenen Tiefpassfiltern beseitigt, die hochohmigen Eingänge des EKG-Geräts beseitigen das Problem des Ausgangswiderstands am Demultiplexer. Ausserdem sind, wie bereits erwähnt, die beiden Eingänge des EKG-Geräts für die EKG-Referenzelektroden ("Fuss rechts, Fuss links") mit der Masse des Empfängerteils verbunden. Teile des Empfängers dieser oben als Zusatz zu einem herkömmlichen EKG-System beschriebenen Vorrichtung können auch in das EKG-Gerät integriert werden, was den gesamten gerätetechnischen Aufwand vermindert. Generell kann der Aufwand für die elektrische Patientensicherheit im Empfangsteil und im EKG-Gerät gegenüber der herkömmlichen Ausbildung, die galvanisch mit dem Patienten verbunden ist, stark reduziert werden, weil die elektromagnetische und insbesondere optische Strecke die galvanische Trennung bewirkt.

In bezug auf die zwei Übertragungsarten über den freien Luftraum oder über einen Lichtleiter ergeben sich für die Erfindung verschiedene Verwendungsmöglichkeiten. Der Senderteil und der Empfängerteil können beide durch Betätigung eines Umschalters auf die eine oder die andere Übertragungsart umgeschaltet und somit für beide Übertragungsarten verwendet werden.

Die Übertragung über den freien Luftraum eignet sich dann besonders, wenn es dem Patienten möglich sein soll, sich ungehindert frei zu bewegen, beispielsweise bei ergonometrischen Messungen.

Die Übertragung über einen Lichtleiter eignet sich besonders für die Anwendung in einem Operationssaal oder in einer Intensivstation, wo die Störung der Übertragung durch Lichtquellen sehr stark sein kann. Bei einem bettlägerigem Patienten kann der Senderteil am Bett befestigt sein, die optische Übertragung erfolgt sehr einfach über eine lösbare Steckverbindung des Lichtleiters am Senderteil und am EKG-Gerät, so dass der Patient schnell und einfach "umsteckbar" ist. Die Übertragung über einen Lichtleiter eignet sich auch sehr gut zur Fernüberwachung von Patienten von einer Wachstation aus, da der Lichtleiter die Signale problemlos, ohne äussere Störeinflüsse und ohne dass eine Verstärkung nötig ist, über Entfernungen von bis zu 50m überträgt.

Grosse Vorteile werden bei einer Verwendung der Erfindung in der Notfallmedizin erreicht. Einem Unfallopfer wird bereits am Unfallort ein Senderteil angelegt, der die EKG-Signale ununterbrochen zum EKG-Gerät zunächst im Krankenwagen, später im Operationssaal und auf Station überträgt.

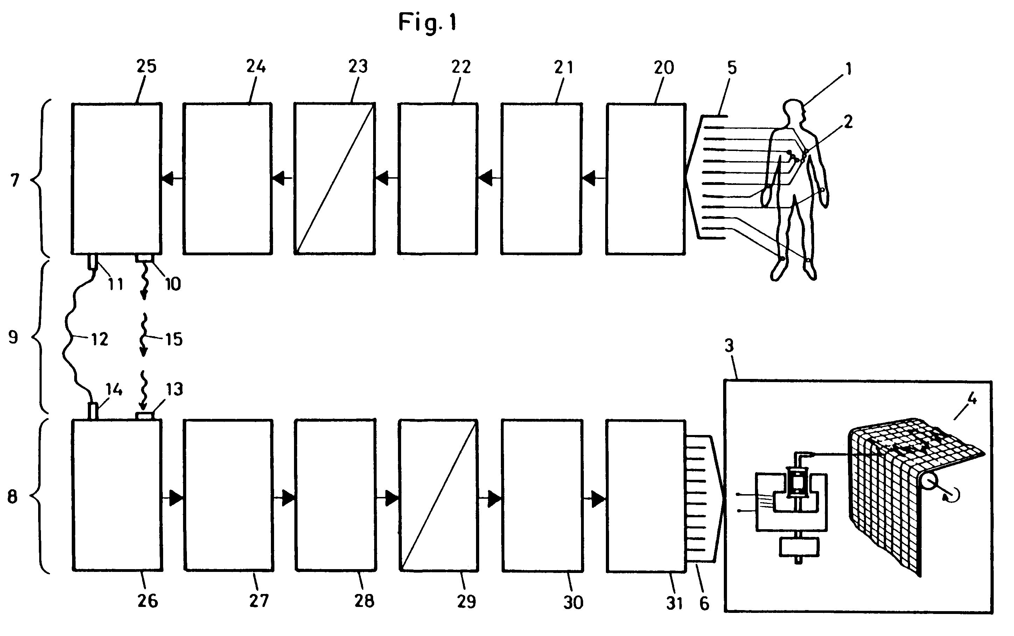

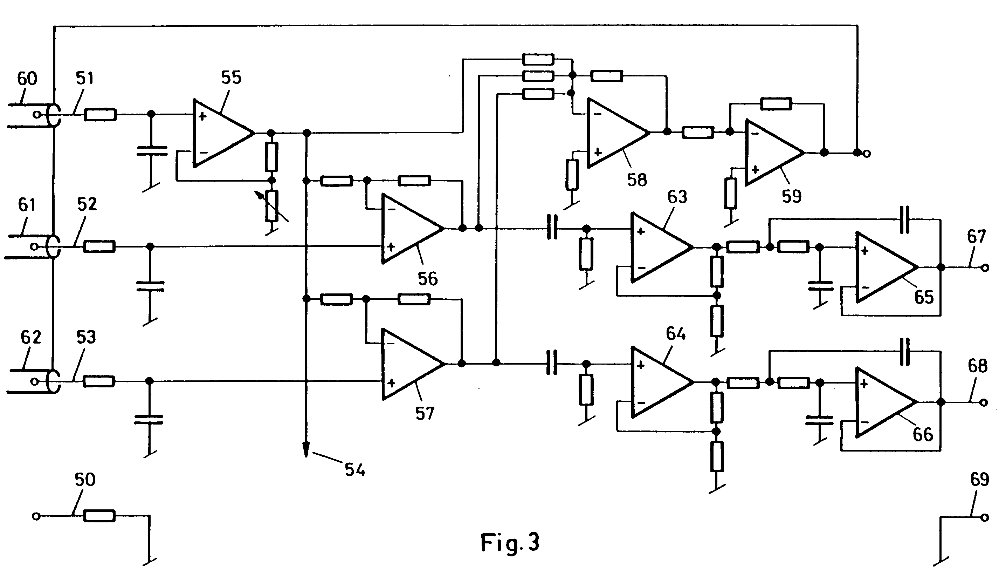

Fig. 1 zeigt ein Blockschaltbild eines gesamten EKG-Systems, das mit einer erfindungsgemässen Vorrichtung versehen ist. In diesem System werden Elektrokardiogramm-Signale (EKG-Signale) von an einem Patienten 1 angebrachten EKG-Elektroden 2 zu einem EKG-Gerät 3 übertragen, das die EKG-Signale verarbeitet und dann aufzeichnet, wie es in 4 symbolisiert wird. Die EKG-Elektroden 2 sind am Patienten 1 an vorbestimmten, in der medizinischen Praxis zum Erhalt der sogenannten Standardableitungen üblich gewordenen Stellen "Arm links, Arm rechts, Fuss links, Fuss rechts, Brustwand Position 1 bis 6" angebracht. Zwei dieser EKG-Elektroden ("Fuss links, Fuss rechts") dienen als Referenz bei der Erfassung der Signale der anderen Elektroden und werden im nachfolgenden als erste ("Fuss links") und zweite ("Fuss rechts") EKG-Referenzelektroden definiert. Die übrigen EKG-Elektroden werden aus Klarheitsgründen als EKG-Signalelektroden definiert.

In einem normalen herkömmlichen System ohne Verwendung der Erfindung bilden der Strang 5 von Ausgangsleitungen der EKG-Elektroden 2 und der Strang 6 von Eingangsleitungen des EKG-Geräts 3 einen und denselben Strang: entsprechend sind die Eingänge des EKG-Geräts 3 den EKG-Elektroden 2 zugeordnet und auch jeweils so bezeichnet ("Arm links, Arm rechts, Brustwand Position 1 bis 6"). Bei Verwendung der Erfindung wird hingegen die erfindungsgemässe Vorrichtung zwischen den Strängen 5 und 6 geschaltet.

Die erfindungsgemässe Vorrichtung umfasst einen Senderteil 7, der im oberen Teil der Fig. 1 durch eine geschwungene Klammer angedeutet ist, sowie einen Empfängerteil 8, der im unteren Teil der Fig. 1 durch eine geschwungene Klammer angedeutet ist. Zwischen dem Senderteil 7 und dem Empfängerteil 8 liegt mindestens eine Lichtstrecke 9, die im mittleren Teil der Fig. 1 durch eine geschwungene Klammer angedeutet ist und nachstehend näher erläutert wird.

Der Senderteil 7 umfasst mindestens einen modulierbaren Lichtsender, in der Ausbildung nach Fig. 1 sind es zwei Lichtsender 10 und 11, die wahlweise oder beide in Betrieb gesetzt werden können. Der Lichtsender 10 kann aus einem oder mehreren Sendeelementen bestehen und sendet moduliertes Licht 15, vorzugsweise Infrarotlicht, in den umliegenden freien Raum. An den Lichtsender 11 kann man ein flexibles Lichtleitkabel 12 mittels einer (nicht dargestellten) lösbaren Steckverbindung anschliessen, der Lichtsender 11 speist dann Licht, vorzugsweise Infrarotlicht, in das angeschlossene Lichtleitkabel 12 ein. Beide Lichtsender 10 und 11 sind elektrooptische Wandler, die fähig sind, ein elektrisches Signal in moduliertes Licht und vorzugsweise Infrarotlicht zu wandeln.

Der Empfängerteil 8 umfasst mindestens einen Lichtempfänger, in der Ausbildung nach Fig. 1 sind es zwei Lichtempfänger 13 und 14, die wahlweise oder beide in Betrieb gesetzt werden können. Der Lichtempfänger 13 kann aus einem oder mehreren Empfangselementen bestehen und empfängt Licht 15, vorzugsweise Infrarotlicht, aus dem umliegenden freien Raum. An den Lichtempfänger 14 kann man das flexible Lichtleitkabel 12 mittels einer (nicht dargestellten) lösbaren Steckverbindung anschliessen, der Lichtempfänger 14 empfängt dann das Licht, vorzugsweise Infrarotlicht, das ihm vom angeschlossenen Lichtleitkabel 12 zugeführt wird. Beide Lichtempfänger 13 und 14 sind optoelektrische Wandler, die fähig sind, die Modulation von Licht und vorzugsweise Infrarotlicht in ein demoduliertes elektrisches Signal zu wandeln.

Somit umfasst die Lichtstrecke 9 wahlweise den freien Luftraum zwischen dem Lichtsender 10 und dem Lichtempfänger 13 oder einen als flexibles Lichtleitkabel 12 ausgebildeten Lichtleiter. Es ist zu verstehen, dass andere Ausbildungen des Lichtleiters zum Beispiel als einzelne Lichtleitfaser mit oder ohne lösbaren Steckverbindungen möglich und verwendbar sind. Es ist erkennbar, dass die Übertragung von Signalen vom Senderteil 7 zum Empfängerteil 8, also die Übertragung von EKG-Signalen von den EKG-Elektroden 2 zum EKG-Gerät 3, jedenfalls optisch, d.h. nicht-elektrisch, und wahlweise immateriell, d.h. drahtlos und kabellos erfolgt. Zwischen den am Patienten 1 angebrachten EKG-Elektroden 2 und dem EKG-Gerät 3 wird keine elektrische Verbindung erstellt.

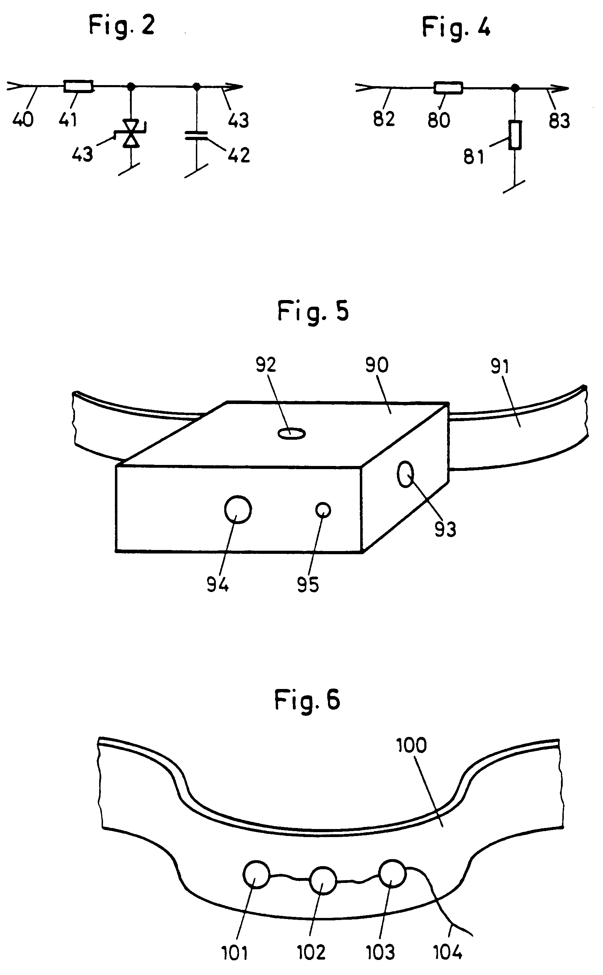

Im Senderteil 7 ist ein Eingangsverstärker 20 vorgesehen, der nachstehend im Zusammenhang mit Fig. 2 und 3 näher erläutert wird. Die von den EKG-Elektroden 2 abgegriffenen EKG-Signalen werden dem Eingangsverstärker 20 werden über den Strang 5 zugeführt. Der Eingangsverstärker 20 weist für jede EKG-Elektrode 2 einen zugeordneten Eingangskanal auf, an den je eine zugeordnete EKG-Elektrode anschliessbar ist, und gewährleistet auf diesen Eingangskanälen den erforderlichen Eingangsschutz mit Hilfe von an sich in EKG-Systemen bekannten Eingangsschutz-Schaltungen.

Fig. 2 zeigt schematisch eine Ausbildung einer solchen Schaltung zum Eingangsschutz, wie sie auf jedem Eingangskanal gleich am Eingang des Eingangsverstärkers 20 vorgesehen ist, d.h. praktisch an den Steckbuchsen, welche am Gehäuse des Eingangsverstärkers 20 die Stecker der Leitungen des Stranges 5 aufnehmen. Die Leitung 40 ist mit einer Leitung des Stranges 5 verbunden. Das aus dem Widerstand 41 und dem Kondensator 42 bestehende RC-Glied mit einer Grenzfrequenz von etwa 3kHz lässt die Nutzsignalanteile ungehindert durch, Hochfrequenzanteile werden hingegen gedämpft. Zur Abschirmung gegen Defibrillationsimpulsen ist eine Spannungsbegrenzung durch das antiseriell geschaltete Zenerdioden-Paar 43 vorgesehen, welche die zu den Verstärkereingängen gelangenden Spannungen begrenzt und die nachfolgenden elektronischen Schaltungen vor Zerstörung schützt. Die solcherweise geschützte Leitung führt zur weiteren Signalverarbeitung im Eingangsverstärker 20, die nun erläutert wird.

Nach dem Eingangsschutz bildet der Eingangsverstärker 20 mittels an sich in EKG-Systemen bekannter Rechenschaltungen für jede EKG-Signalelektrode die Differenz zwischen dem daran abgenommenen EKG-Signal und dem an der ersten EKG-Referenzelektrode ("Fuss links") abgenommenen EKG-Signal (vgl. näheres nachstehend im Zusammenhang mit Fig. 3). Folglich ist auch jede so gebildete Signaldifferenz einer EKG-Signalelektrode zugeordnet. Der Ausgang der Eingangsschutz-Schaltung, die der zweiten EKG-Referenzelektrode ("Fuss rechts") zugeordnet ist, wird dabei mit einer gemeinsamen Masseleitung der Rechenschaltungen verbunden, um die Masse der Rechenschaltungen und allgemein des Eingangsverstärkers 20 und des Senderteils 7 dem patientenseitigen Massepotential anzugleichen.

Fig. 3 zeigt schematisch eine entsprechende Schaltung zur Vorverstärkung und zur Differenzbildung, die der Schaltung zum Eingangsschutz wie erwähnt nachgeschaltet ist. Jeweils über die zugeordnete Schaltung zum Eingangsschutz (beispielsweise eine solche, wie sie im Zusammenhang mit Fig. 2 beschrieben wurde) ist die Leitung 50 mit der zweiten EKG-Referenzelektrode ("Fuss rechts") und die Leitung 51 mit der ersten EKG-Referenzelektrode ("Fuss links") verbunden. Die Leitungen 52 und 53 sind mit über die jeweils zugeordnete Schaltung zum Eingangsschutz mit je einer EKG-Signalelektrode und stehen in Fig. 3 stellvertretend für alle anderen, nicht dargestellten EKG-Signalelektroden, was auch mit dem Pfeil 54 angedeutet wird. Jedes EKG-Signal wird dem nichtinvertierenden Eingang eines zugeordneten Rechenverstärkers 55, 56, 57 usw. zugeführt, da dieser Eingang hochohmig ist. Zur richtigen Bildung der gewünschten Differenzen müssen alle EKG-Signale in ihrem jeweiligen Rechenverstärker 55, 56, 57 usw. um den gleichen und richtigen Faktor verstärkt werden: dies wird einerseits durch die Rückkopplungswiderstände an den Rechenverstärkern 55, 56, 57 usw. und andererseits durch Einstellung der Verstärkung mit dem variablen Widerstand in der Rückkopplung am Rechenverstärker 55 erreicht. Der Ausgang des Rechenverstärkers 55, welcher der ersten EKG-Referenzelektrode ("Fuss links") zugeordnet ist, ist mit den invertierenden Eingängen der Rechenverstärker 56, 57 usw., die den EKG-Signalelektroden zugeordnet sind, über einen Widerstand der entsprechenden Rückkopplungskette verbunden (der Pfeil 54 symbolisiert die entsprechende Verbindung mit den nicht dargestellten Rechenverstärkern).

Der Ausgang eines jeden Rechenverstärkers 55, 56, 57 ist auch noch über je einen Widerstand mit dem invertierenden Eingang des Rechenverstärkers 58 verbunden, so dass der Ausgang dieses Rechenverstärkers 58 ein Signal führt, das den Mittelwert der dadurch ausgewählten EKG-Signale darstellt. Weil die Summenbildung an diesem Rechenverstärker 58 invertierend ist, ist noch ein weiterer Rechenverstärker 59 zur Inversion des erhaltenen Signals vorgesehen, das den Kabelabschirmungen 60, 61, 62 usw. zugeführt wird, um deren Potential mitzuführen ("Boot-strapping"). Die Rechenverstärker 64, 65 usw. sind als nichtinvertierende spannungsgesteuerte Spannungsquellen geschaltet und dienen der Gleichspannungs-Trennung und Impedanzwandlung. Ihr Ausgang führt zu den Rechenverstärkern 65, 66 usw., die zum Zwecke der "Anti-Aliasing"-Filterung als Butterworthfilter 2. Ordnung geschaltet sind. Diese "Anti-Aliasing"-Filter liefern die weiter zu verarbeitenden EKG-Signale auf Ausgangsleitungen 67, 68 usw. mit Bezug auf die Masseleitung 69.

Die Signaldifferenzen aus dem Eingangsverstärker 20 werden also über je einen "Anti-Aliasing"-Filter, der in Fig. 1 im Block 21 schematisch zusammengefasst dargestellt ist und dessen Funktion im vorstehenden bereits erläutert wurde, einem Multiplexer 22 zugeführt, der sie seinerseits einem Analog/digital-Wandler 23 weitergibt. Die im Analog/digital-Wandler 23 gewonnenen Digitalwerte werden in einem Codierer 24 im Manchester-Code oder als FSK-Modulation codiert. Der Zweck und die Vorteile dieser Codierung wurden bereits im vorstehenden erläutert. Gegebenenfalls wird hier auch, mit Hilfe einer geeigneten Schaltung, eine Fehlerschutz-Zusatzinformation beigefügt, z.B. zur zyklischen Redundanzprüfung ("cyclic redundancy check" = CRC) oder zur Paritätsprüfung nach dem Hamming-Code.

Vom Ausgang des Codierers 24 werden die codierten Daten einem Modulationseingang einer Lichtsendeeinheit 25, welche die Lichtsender 10 und 11 und deren Speise- und Steuerschaltungen umfasst. Das von den Lichtsendern 10 und 11 elektrooptisch gewandelte Modulationssignal entspricht somit den im Eingangsverstärker 20 gebildeten Signaldifferenzen, wobei dieses Modulationssignal aufgrund der vorangehenden Signalverarbeitung vom Gleichspannungsanteil und vom Brumm der EKG-Signale weitgehend befreit ist ("common mode rejection").

Im Empfängerteil 8 ist eine Lichtempfangseinheit 26 vorgesehen, welche die Lichtempfänger 13 und 14 und deren Speise- und Ausgangsschaltungen (Transimpedanzwandlung von kleinen Strömen in verarbeitbare Spannungen) umfasst. Das von den Lichtempfängern 13 und 14 optoelektrisch gewandelte Signal wird somit in der Lichtempfangseinheit 26 demoduliert und das gewonnene Modulationssignal wird in einer Schaltung 27 zur Weiterverwendung aufbereitet: entsprechend dem Frequenzspektrum des Manchester-Codes bzw. der FSK-Modulation werden die kontinuierlichen und niederfrequenten Anteile des Modulationssignals (inklusiv 50-Hz-Brumm und 100-Hz-Störung durch Leuchtstoffröhren und deren Oberschwingungen) sowie die hochfrequenten Rausch- und Störanteile mit Filtern eliminiert, gleichzeitig erfolgt eine Verstärkung auf einen weiterverwendbaren Signalwert. Eine solche Signalaufbereitung ist an sich bekannt und braucht nicht näher beschrieben zu werden.

Das in der Schaltung 27 aufbereitete, an einem Modulationsausgang derselben anstehende Modulationssignal wird in einem Decodierer 28 zugeführt, welches die im Codierer 24 im Manchester-Code oder als Frequenzsprung-Modulation verarbeiteten Digitalwerte wiederherstellt. Gegebenenfalls wird hier auch, mit Hilfe einer geeigneten Schaltung, eine Korrektur mit Hilfe der im Sendeteil beigefügten Fehlerschutz-Zusatzinformation vorgenommen.

Die so gewonnenen Digitalwerte werden einem Digital/analog-Wandler 29 zugeführt, um die Analogsignale wiederzugewinnen. Bei der dabei erfolgenden Glättung der Analogsignale (Tiefpass-Filterung) erfolgt eine Interpolation der Stützwerte, welche die Korrektur der von der Multiplexierung verursachten Zeitverschiebung bewirkt. Somit werden die Analogsignale wieder zeitgleich und es wird dadurch möglich, sie dem EKG-Gerät zeitgleich zuzuführen.

Die so gewonnenen und verarbeiteten Analogsignale werden einem Demultiplexer 30 zugeführt. Dieser ist mit Ausgängen versehen, von denen jeder einer der im Eingangsverstärker 20 des Senderteil 7 gebildeten Signaldifferenzen zugeordnet ist. Die Signale aus diesen Ausgängen des Demultiplexers 30 werden noch in einer Schaltung 31 auf solche Werte abgeschwächt, die den normalen Werten der reellen, direkt an EKG-Elektroden 2 abgegriffenen EKG-Signale entsprechen, was ermöglicht, sie über den Strang 6 direkt den entsprechenden Eingängen des EKG-Geräts 3 einzuspeisen. Da aber nur die EKG-Signale aus den EKG-Signalelektroden über die Lichtstrecke 9 übertragen und über den Strang 6 den entsprechenden Eingängen des EKG-Geräts ("Arm links, Arm rechts, Brustwand Position 1 bis 6") zugeführt werden, leistet das Potential an den übrigen Eingängen des EKG-Geräts ("Fuss rechts, Fuss links") keinen nützlichen Beitrag, daher sind diese beiden letzten Eingänge (auf nicht dargestellte Weise) mit der Masse des EKG-Geräts verbunden.

Wie ersichtlich erfolgt die Übertragung der EKG-Signale über die Lichtstrecke 9 seriell als Einweg-Übertragung Daten zum EKG-Gerät hin, wobei der Codierer 24 und der Decodierer 28 beide zur Verarbeitung des Manchester-Codes oder der Frequenzsprung-Modulation bestimmt und ausgebildet sind.

Fig. 4 zeigt eine schematisch angegebene Ausbildung einer im Empfängerteil der erfindungsgemässen Vorrichtung verwendeten Ausgangsschaltung, die im Block 31 der Fig. 1 symbolisch dargestellt worden ist. Da die übertragenen und danach decodierten Signale bereits den gewünschten Signalen entsprechen, ist nur mehr deren Wert so einzustellen, dass das EKG-Gerät keinen Unterschied "bemerkt", ob nun der Patient direkt über die üblichen Kabel oder über die erfindungsgemässe Vorrichtung daran angeschlossen ist. Dies erfordert nur eine lineare Abschwächung, die mit dem dargestellten Ohmschen Spannungsteiler mit den Widerständen 80, 81 erreicht wird. Die Eingangsleitung 82 dieser Schaltung ist mit einem der Ausgänge des Demultiplexers 30 und die Ausgangsleitung 83 über eine Leitung des Stranges 6 mit einem der entsprechenden Eingängen des EKG-Geräts ("Arm links, Arm rechts, Brustwand Position 1 bis 6") verbunden.

Der Senderteil 7 kann in einer Variante als selbständiges einteiliges Gerät ausgebildet sein, das vom Patienten beispielsweise am Gürtel, auf dem Rücken oder umgehängt getragen werden kann.

Fig. 5 zeigt schematisch und perspektivisch ein Gehäuse 90 eines solchen Senderteils 7, das mit einem Gurt 91 umgeschnallt getragen werden kann. Dieses Gehäuse 90 ist mit einem oder mehreren, beispielsweise vier Lichtsendern 92, 93, 94 (der vierte ist in Fig. 5 nicht sichtbar) versehen. Diese Lichtsender sind beispielsweise Infrarotsender und sind an dem Patienten nicht zugewendeten Seiten des Senderteils 7 bzw. dessen Gehäuses 90 angeordnet, beispielsweise je einer an der oberen und an den drei freistehenden seitlichen Wandungen des Gehäuses 90. Am Gehäuse 90 ist noch eine Steckdose 95 zum Anschliessen eines flexiblen Lichtleitkabels 12 vorgesehen.

Der Senderteil 7 kann in einer anderen Variante als selbständiges zweiteiliges Gerät ausgebildet sein. Der eine Teil ist ein Betriebsteil, der vom Patienten beispielsweise am Gürtel, in der Tasche oder umgehängt getragen werden kann. Der andere Teil ist ein Lichtsender-Tragteil, der vom Patienten beispielsweise als Stirnband getragen werden kann.

Fig. 6 zeigt schematisch und perspektivisch ein solches Stirnband 100, das hier drei Lichtsender 101, 102, 103 an seiner dem Patienten nicht zugewendeten Seite aufweist. Diese Lichtsender 101, 102, 103 sind untereinander und mit dem Betriebsteil durch das dünne Kabel 104 verbunden. Das Kabel 104 kann den Patient kaum stören, denn seine beiden Enden sind an Teilen angeschlossen, die der Patient mit sich führt. Der Betriebsteil ist nicht dargestellt worden, weil er in einer möglichen Ausbildung dem in Fig. 5 dargestellten Senderteil 7 sehr ähnlich ist, sein Gehäuse ist beispielsweise gleich dem in Fig. 5 dargestellten Gehäuse 90 ohne die Lichtsender 92, 93, 94.

Zum Empfang der von diesen Lichtsendern ausgehenden Strahlung sind (auf nicht dargestellte Weise) mindestens ein und vorzugsweise mehrere Lichtempfänger an einem bzw. mehreren festen Stellen eines den Patienten umgebenden Raumes angeordnet, beispielsweise mitten auf zwei oder drei Wänden des Raumes, in welchem sich der Patient befindet, oder auch noch am EKG-Gerät selbst.

**Patentansprüche**

1. Biotelemetrie-Verfahren zur Übertragung von als bioelektrischen Potentialdifferenzen erzeugten Elektrokardiogramm-Signalen (EKG-Signalen) zu einem zwölf Standardableitungen liefernden EKG-Gerät, wobei man mindestens zehn EKG-Elektroden am Patienten auf zum Erhalt der Standardableitungen übliche Weise ("Arm links, Arm rechts, Fuss links, Fuss rechts, Brustwand Position 1 bis 6") anbringt, wobei die Übertragung der EKG-Signale als optische Einweg-Übertragung vom Patienten zum EKG-Gerät hin erfolgt, während keine elektrische Verbindung zwischen den Elektroden und dem EKG-Gerät besteht, und die im EKG-Gerät aufzuzeichnenden Potentialdifferenzen aus der Differenz zwischen zwei übertragenen Signalen gewonnen werden,
**dadurch gekennzeichnet, dass man**
zwei bestimmte EKG-Elektroden ("Fuss links, Fuss rechts") als erste ("Fuss links") und zweite ("Fuss rechts") EKG-Referenzelektroden und die übrigen EKG-Elektroden ("Arm links, Arm rechts, Brustwand Position 1 bis 6") als EKG-Signalelektroden definiert,
die zweite EKG-Referenzelektrode ("Fuss rechts") zur Bestimmung eines patientenseitigen Massepotentials verwendet,
für jede EKG-Signalelektrode die Differenz zwischen dem daran abgenommenen EKG-Signal und dem an der ersten EKG-Referenzelektrode ("Fuss links") abgenommenen EKG-Signal bildet und somit ein zu übertragendes Signal erzeugt, das vom gemeinsamen Gleichspannungsanteil und vom Brumm befreit ist ("common mode rejection"),
die zu übertragenden Signale einweg-seriell überträgt, indem man sie multiplexiert, das Multiplex-Signal analog/digital wandelt und danach in ein codiertes Signal umsetzt, das gegebenenfalls mit Fehlerschutz-Zusatzinformation versehen wird, mit dem codierten Signal einen Lichtstrahl moduliert, den Lichtstrahl sendet, überträgt und empfängt, den empfangenen Lichtstrahl demoduliert, die gewonnene Modulation decodiert, das decodierte Signal gegebenenfalls nach Vornahme einer Korrektur mit Hilfe der Fehlerschutz-Zusatzinformation digital/analog wandelt und demultiplexiert, um übertragene EKG-Signale zu erhalten,
und die übertragenen EKG-Signale den entsprechenden Eingängen ("Arm links, Arm rechts, Brustwand Position 1 bis 6") des EKG-Geräts zuführt, während man die übrigen Eingänge des EKG-Geräts ("Fuss rechts, Fuss links") mit einer Masse des EKG-Geräts verbindet.

2. Verfahren nach Anspruch 1, dadurch gekennzeichnet, dass die Übertragung über einen Lichtleiter geführt wird.

3. Verfahren nach Anspruch 1, dadurch gekennzeichnet, dass die analog/digital gewandelten Signale im Manchester-Code oder als Frequenzsprung-Modulation codiert werden.

4. Verfahren nach Anspruch 1, dadurch gekennzeichnet, dass die analog/digital gewandelten Signale mit einer Fehlerschutz-Zusatzinformation versehen werden.

5. Vorrichtung zur Durchführung des Verfahrens nach Anspruch 1 in einem EKG-System, das mindestens zehn an einem Patienten angebrachte EKG-Elektroden ("Arm links, Arm rechts, Fuss links, Fuss rechts, Brustwand Position 1 bis 6") und ein EKG-Gerät für zwölf Standardableitungen umfasst, wobei zwei bestimmte EKG-Elektroden ("Fuss links, Fuss rechts") als erste ("Fuss links") und zweite ("Fuss rechts") EKG-Referenzelektroden und die übrigen EKG-Elektroden als EKG-Signalelektroden definiert sind, mit:
einem Senderteil, einem Empfängerteil und mindestens einer dazwischenliegenden Lichtstrecke,
an sich in EKG-Systemen bekannten Eingangsschutz-Schaltungen, an die je eine zugeordnete EKG-Elektrode anschliessbar ist,
an sich in EKG-Systemen bekannten Rechenschaltungen zur Gewinnung der Differenz zwischen Signalen aus zwei Eingangsschutz-Schaltungen, von denen die eine einer EKG-Signalelektrode ("Arm links, Arm rechts, Brustwand Position 1 bis 6") und die andere der ersten EKG-Referenzelektrode ("Fuss links") zugeordnet ist, so dass auch jede Signaldifferenz einer EKG-Signalelektrode zugeordnet ist, mindestens einen modulierbaren Lichtsender und mindestens einen Lichtempfänger mit einem Ausgang zur Abgabe der Modulation eines empfangenen Lichtstrahls,
**gekennzeichnet durch**
eine Verbindung des Ausgangs der Eingangsschutz-Schaltung, die der zweiten EKG-Referenzelektrode ("Fuss rechts") zugeordnet ist, mit einer gemeinsamen Masseleitung der Rechenschaltungen,

einen im Senderteil angeordneten Multiplexer für die gewonnenen Signaldifferenzen, dessen Ausgang an einen Modulationseingang des Lichtsenders über die Reihenschaltung eines Analog/Digital-Wandlers, eines Codierers und gegebenenfalls einer Schaltung zum Zufügen von Fehlerschutz-Zusatzinformation angeschlossen ist, und

einen im Empfängerteil angeordneten Demultiplexer mit einem Eingang, der an den Ausgang des bzw. der Lichtempfänger gegebenenfalls über eine Schaltung zur Vornahme einer Korrektur mit Hilfe der Fehlerschutz-Zusatzinformation und dann über die Reihenschaltung eines Decodierers und eines Digital/Analog-Wandlers angeschlossen ist, und mit Ausgängen, von denen jeder einer der im Senderteil gebildeten Signaldifferenzen zugeordnet ist und ein Signal führt, das einem entsprechenden Eingang ("Arm links, Arm rechts, Brustwand Position 1 bis 6") des EKG-Geräts zuführbar ist.

6. Vorrichtung nach Anspruch 5, dadurch gekennzeichnet, dass der Senderteil als selbständiges Gerät ausgebildet ist, das vom Patienten tragbar ist.

7. Vorrichtung nach Anspruch 6, dadurch gekennzeichnet, dass mindestens ein und vorzugsweise mehrere Lichtsender an mindestens einer dem Patienten nicht zugewendeten Seite des Senderteils und mindestens ein und vorzugsweise mehrere Lichtempfänger an einem bzw. mehreren festen Stellen eines den Patienten umgebenden Raumes angeordnet sind.

8. Vorrichtung nach Anspruch 5, dadurch gekennzeichnet, dass der Senderteil als selbständiges zweiteiliges Gerät mit einem Betriebsteil und einem Lichtsender-Tragteil ausgebildet ist, wobei die beiden Teile vom Patienten tragbar sind und der Lichtsender-Tragteil mindestens ein und vorzugsweise mehrere Lichtsender an mindestens einer dem Patienten nicht zugewendeten Seite aufweist, während mindestens ein und vorzugsweise mehrere Lichtempfänger an einem bzw. mehreren festen Stellen eines den Patienten umgebenden Raumes angeordnet sind.

## Claims

1. A biotelemetric method for the transmission of electrocardiogram signals (ECG signals) generated as bioelectric potential differences to an an ECG device that supplies the twelve standard derivations, at least ten ECG electrodes being applied on the patient in the customary way for obtaining the standard derivations ("left arm, right arm, left foot, right foot, chest position 1 to 6"), the transmission of the ECG signals being performed as optical one-way transmission from the patient to the ECG device while there is no electric connection between the electrodes and the ECG device, and the potential differences to be recorded in the ECG device being obtained from the difference between two transmitted signals,
**characterized in that**
two specific ECG electrodes ("left foot, right foot") are defined as first ("left foot") and second ("right foot") ECG reference electrodes and the remaining ECG electrodes ("left arm, right arm, chest position 1 to 6") as ECG signal electrodes,

the second ECG reference electrode ("right foot") is used for the determination of a ground potential at the patient's end,

for each ECG signal electrode the difference between the ECG signal obtained from it and the ECG signal obtained at the first ECG reference electrode ("left foot") is formed and thus a signal is produced that is freed from the common direct voltage content and from the hum ("common mode rejection"),

the signals to be transmitted are transmitted in a one-way serial manner by multiplexing them, converting the multiplex signal from analog to digital, and subsequently into an encoded signal to which, optionally, additional information for error protection is added, with the encoded signal a light beam is modulated, the light beam is sent, transmitted and received, the received light beam is demodulated, the obtained modulation decoded, optionally, the decoded signal is corrected by means of the additional information for error protection, converted from digital to analog, and demultiplexed in order to obtain transmitted ECG signals,

and the transmitted ECG signals are fed to the corresponding inputs ("left arm, right arm, chest position 1 to 6") of the ECG device ("right foot, left foot"), while the remaining inputs of the ECG device ("right foot, left foot") are connected with a ground terminal of the ECG device.

**2.** A biotelemetric method according to claim 1, **characterized in that** the transmission is performed optically via an optical fibre cable.

**3.** A biotelemetric method according to claim 1, **characterized in that** the signals converted from analog to digital are encoded in the Manchester code or as frequency shift keying.

**4.** A biotelemetric method according to claim 1, **characterized in that** the signals converted from analog to digital are equipped with additional information for error protection.

**5.** A device for performing the method of claim 1 in an ECG system comprising at least ten ECG electrodes applied on a patient ("left arm, right arm, left foot, right foot, chest position 1 to 6") and an ECG device for twelve standard derivations, two specific ECG electrodes ("left foot, right foot") being defined as first ("left foot") and second ("right foot") ECG reference electrodes and the remaining ECG electrodes as ECG signal electrodes, having

a transmitting unit, a receiving unit, and at least one optical link in between,

input protection circuits known per se in ECG systems to which one assigned ECG electrode each can be connected,

computing circuits known per se in ECG systems for obtaining the difference between signals from two input protection circuits, one of which is assigned to an ECG signal electrode ("left arm, right arm, chest position 1 to 6") and the other to the first ECG reference electrode ("left foot") so that also each signal difference is assigned to an ECG signal electrode,

at least one modulatable optotransmitter and at least one optoreceiver having an output for delivering the modulation of a received light beam,

**characterized by**

a connection of the output of the input protection circuit that is assigned to the second ECG reference electrode ("right foot"), with a common ground lead for the computing circuits,

a multiplexer for the obtained signal differences, arranged in the transmitting unit and whose output is linked to a modulation input of the optotransmitter via the connection in series of an analog-to-digital converter, an encoder, and, optionally, a circuit for adding additional information for error protection, and

a demultiplexer arranged in the receiving unit and having an input linked to the output of the optoreceiver(s), optionally via a circuit for correction by means of the additional information for error protection, and then via the connection in series of a decoder and a digital-to-analog converter, and having outputs, each of which is assigned to the signal differences formed in the transmitting unit and carrying a signal that can be fed to a corresponding input ("left arm, right arm, chest position 1 to 6") of the ECG device.

**6.** A device according to claim 5, **characterized in that** the transmitting unit is designed as independent device that may be carried by the patient.

**7.** A device according to claim 6, **characterized in that** at least one and preferably several optotransmitters are arranged on at least one side of the transmitting unit not facing the patient, and at least one and preferentially several optoreceivers are arranged at one or several fixed locations of a room surrounding the patient.

**8.** A device according to claim 5, **characterized in that** the transmitting unit is designed as independent two-part device with an operating unit and an optotransmitter portable unit, both units capable of being carried by the patient and possessing at least one and preferentially several optotransmitters on at least one side not facing the patient, while at least one and preferentially several optoreceivers are arranged in one or several fixed locations of a room surrounding the patient.

**Revendications**

**1.** Procédé de biotélémétrie pour la transmission de signaux d'électrocardiogramme (signaux ECG) obtenus sous forme de différences de potentiels bioélectriques, à un appareil ECG procurant douze dérivations standard, dans lequel on applique au moins dix électrodes ECG sur le patient de la manière habituelle ("bras gauche, bras droit, pied gauche, pied droit, paroi thoracique positions 1 à 6") pour obtenir les dérivations standard, dans lequel la transmission des signaux ECG a lieu sous forme d'une transmission optique unidirectionnelle du patient à l'appareil ECG, tandis qu'il n'existe aucune liaison électrique entre les électrodes et l'appareil ECG, et on obtient les différences de potentiels à enregistrer dans l'appareil ECG à partir de la différence entre deux signaux transmis,

**caractérisé en ce que**

on définit deux électrodes ECG déterminées ("pied gauche, pied droit") comme première électrode de référence ECG ("pied gauche") et comme seconde électrode de référence ECG ("pied droit"), et les autres électrodes ECG ("bras gauche, bras droit, paroi thoracique positions 1 à 6") comme électrodes collectrices ECG,

on utilise la seconde électrode de référence ECG ("pied droit") pour déterminer un potentiel de masse côté patient,

on établit, pour chaque électrode collectrice ECG, la différence entre le signal ECG prélevé de cette dernière et le signal ECG prélevé de la première électrode de référence ECG ("pied gauche"), et ainsi, on obtient un signal à transmettre, qui est libéré de la composante commune de tension continue et du ronflement ("common mode rejection" = réjection du mode commun),

on procède à la transmission unidirectionnelle en série des signaux à transmettre en les soumettant à un multiplexage, en soumettant le signal multiplexé à une conversion analogique/numérique et en le transformant ensuite en un signal codé que l'on munit éventuellement d'une information supplémentaire pour la protection contre les erreurs, en modulant un rayon lumineux avec le signal codé, en émettant, en transmettant et en recevant le rayon lumineux, en démodulant le rayon lumineux reçu, en décodant la modulation obtenue, en soumettant à une conversion numérique/analogique et à un démultiplexage le signal décodé éventuellement après avoir procédé à une correction à l'aide de l'information supplémentaire pour la protection contre les erreurs, pour obtenir les signaux ECG transmis,

et on achemine les signaux ECG transmis aux entrées correspondantes ("bras gauche, bras droit, paroi thoracique positions 1 à 6") de l'appareil ECG, tandis que l'on relie les autres entrées de l'appareil ECG ("pied droit, pied gauche") à une masse de l'appareil ECG.

2. Procédé selon la revendication 1, caractérisé en ce qu'on fait passer la transmission par un câble fibre optique.

3. Procédé selon la revendication 1, caractérisé en ce que les signaux ayant subi une conversion analogique/numérique sont codés dans le code Manchester ou sous forme d'une modulation à saut de fréquence.

4. Procédé selon la revendication 1, caractérisé en ce que les signaux ayant subi une conversion analogique/numérique sont munis d'une information supplémentaire pour la protection contre les erreurs.

5. Dispositif pour la mise en oeuvre du procédé selon la revendication 1, dans un système ECG qui comprend au moins dix électrodes ECG placées sur un patient ("bras gauche, bras droit, pied gauche, pied droit, paroi thoracique positions 1 à 6") et un appareil ECG pour douze dérivations standard, dans lequel deux électrodes ECG déterminées ("pied gauche, pied droit") sont définies comme première électrode de référence ("pied gauche") et comme seconde électrode de référence ("pied droit"), et les autres électrodes ECG sont définies comme électrodes collectrices ECG, comprenant :

une partie émettrice, une partie réceptrice et au moins un trajet lumineux disposé entre elles,

des circuits protecteur d'entrée connus en soi dans des systèmes ECG, auxquels peut se raccorder chaque fois une électrode ECG correspondante,

des circuits de calcul connus en soi dans des systèmes ECG pour obtenir la différence entre des signaux issus de deux circuits protecteur d'entrée, dont l'un est attribué à une électrode collectrice ECG ("bras gauche, bras droit, paroi thoracique, positions 1 à 6") et dont l'autre est attribué à la première électrode de référence ECG ("pied gauche") de telle sorte que chaque différence de signal soit également attribuée à une électrode collectrice ECG, au moins un émetteur de lumière modulable et au moins un récepteur de lumière muni d'une sortie pour l'émission de la modulation d'un rayon lumineux reçu, **caractérisé par**

une liaison de la sortie du circuit protecteur d'entrée qui est attribué à la seconde électrode de référence ECG ("pied droit"), à un câble commun de mise à la masse des circuits de calcul,

un multiplexeur disposé dans la partie émettrice, pour les différences de signaux obtenues, dont la sortie est raccordée à une entrée de modulation de l'émetteur de lumière via le montage en série d'un convertisseur analogique/numérique, d'un codeur et éventuellement d'un circuit pour ajouter une information supplémentaire pour la protection contre les erreurs, et

un démultiplexeur disposé dans la partie réceptrice, comprenant une entrée qui est raccordée à la sortie du, respectivement des récepteurs de lumière éventuellement en passant par un circuit permettant de procéder à une correction à l'aide de l'information supplémentaire pour la protection contre les erreurs puis en passant par le montage en série d'un décodeur et d'un convertisseur numérique/analogique, et comprenant des sorties dont chacune est attribuée à une des différences de signaux établies dans la partie émettrice et délivre un signal qui peut être acheminé à une entrée correspondante ("bras gauche, bras droit, paroi thoracique, positions 1 à 6") de l'appareil ECG.

6. Dispositif selon la revendication 5, caractérisé en ce que la partie émettrice est réalisée en un appareil autonome qui peut être porté par le patient.

7. Dispositif selon la revendication 6, caractérisé en ce qu'au moins un, de préférence plusieurs émetteurs de lumière sont disposés contre au moins un des côtés de la partie émettrice qui n'est pas tourné vers le patient, et au moins un, de préférence plusieurs récepteurs de lumière sont disposés à un, respectivement à plusieurs endroits fixes d'un espace entourant le patient.

8. Dispositif selon la revendication 5, caractérisé en ce que la partie émettrice est réalisée sous forme d'un appareil autonome en deux parties, comprenant une partie de fonctionnement et une partie de support de l'émetteur de lumière, les deux parties pouvant être portées par le patient, et la partie de support de l'émetteur de lumière présentant au moins un, de préférence plusieurs émetteurs de lumière sur au moins un côté qui n'est pas tourné vers le patient, tandis qu'au moins un, de préférence plusieurs récepteurs de lumière sont disposés à un, respectivement plusieurs endroits fixes d'un espace entourant le patient.

Fig.1

Fig.3

19

Fig. 2

Fig. 4

Fig. 5

Fig. 6